# EUROPÄISCHE PATENTSCHRIFT

(11) **EP 4 406 508 B1**
(45) Veröffentlichungstag und Bekanntmachung des Hinweises auf die Patenterteilung: **27.05.2026**
(21) Anmeldenummer: 24174127.1
(22) Anmeldetag: 12.03.2016
(51) Int. Cl.: G02B 21/00, G02B 23/24, A61B 90/00, A61B 1/00, A61B 1/05, A61B 5/00, A61B 90/30, H04N 13/25

(54) **VORRICHTUNG ZUM AUFNEHMEN EINES BILDES EINES OBJEKTFELDS AN EINEM MENSCHLICHEN ODER TIERISCHEN KÖRPER**
DEVICE FOR RECORDING AN IMAGE OF AN OBJECT FIELD ON A HUMAN OR ANIMAL BODY
DISPOSITIF POUR PRENDRE UNE IMAGE D'UN CHAMP D'OBJET SUR UN CORPS HUMAIN OU ANIMAL

(30) Priorität: 24.03.2015 DE 102015003681
(43) Veröffentlichungstag der Anmeldung: 31.07.2024
(62) Teilanmeldung aus: 16000595.5
(73) Patentinhaber: Karl Storz SE & Co. KG, 78532 Tuttlingen (DE)
(72) Erfinder: Schwarz, Peter, 78532 Tuttlingen (DE); Graf, Christian, 78532 Tuttlingen (DE)
(74) Vertreter: Weidner Stern Jeschke

(56) Entgegenhaltungen:
- EP-A1- 2 108 306
- WO-A1-2012/041445
- WO-A2-03/043489

## Beschreibung

Die vorliegende Erfindung betrifft eine Vorrichtung zum Aufnehmen eines Bildes eines Objektfelds an einem menschlichen oder tierischen Körper nach dem Oberbegriff von Anspruch 1.

Aus US 7,649,685 B2 ist eine Anordnung für ein Stereomikroskop mit einer Beleuchtungsvorrichtung bekannt, deren Licht in einem regulierbaren spektralen Bereich liegt. In einen Beobachtungsstrahlengang kann ein Filter mittels einer elektromechanischen Bewegungseinrichtung eingeschwenkt oder eingeschoben werden. Um Filter in die Beleuchtungsstrahlengänge einzubringen, sind ähnliche elektromechanische Bewegungseinrichtungen vorgesehen. Gemäß US 8,614,851 B2 können bei einem Fluoreszenz-Operations-Stereomikroskop zur steuerbaren Abschwächung im Anregungs-Wellenlängenbereich Beobachtungsfilter in den Beobachtungsstrahlengang eingeschwenkt oder eingeschoben werden.

Die Patentschrift DE 10 2006 004 232 C5 offenbart ein Mikroskopiesystem zur Beobachtung von Fluoreszenzen verschiedener Fluoreszenzfarbstoffe in einer Objektebene, wobei das Mikroskopiesystem ein Beleuchtungssystem und ein Beobachtungssystem umfasst. In einem ersten und einem zweiten Beobachtungsstrahlengang zur optischen Abbildung des Objekts ist jeweils ein erster bzw. zweiter Beobachtungsfilterträger angeordnet, die jeweils als drehbar gelagerte Aluminiumscheibe ausgebildet sind, welche jeweils zwei unterschiedliche Transmissionsfilter tragen und eine Öffnung aufweisen. Mittels zweier Antriebe können der erste und der zweite Beobachtungsfilterträger jeweils um eine zur optischen Achse des betreffenden Beobachtungsstrahlengangs parallele Achse gedreht werden, um entsprechend einem jeweils gewählten Betriebszustand entsprechende Transmissionsfilter in dem ersten bzw. zweiten Beobachtungsstrahlengang anzuordnen.

In WO 2012/041445 A1 ist eine Sensoreinheit für ein Stereoendoskop mit zwei Bildsensoren offenbart, die symmetrisch zu einer zentralen Rotationsachse der Sensoreinheit angeordnet sind und die jeweils um eine durch den Bildsensor verlaufende eigene Rotationsachse drehbar sind, wobei die Drehungen der Bildsensoren miteinander gekoppelt sind. Ein Stereoendoskopiesystem umfasst ein Stereoendoskop, das einen Schaft mit zwei getrennten optischen Einheiten aufweist, die zur Änderung der Blickrichtung um eine zentrale Rotationsachse des Stereoendoskops gedreht werden, wobei die zentrale Rotationsachse des Stereoendoskops und die zentrale Rotationsachse der Sensoreinheit zusammenfallen. Durch eine entsprechende Drehung der Sensoreinheit wird es ermöglicht, die Horizontlage der Bildsensoren konstant zu halten, auch wenn die Blickrichtung des Endoskops geändert wird.

Aus der Patentschrift DE 199 03 437 C1 ist eine Vorrichtung zum Ein- und Ausschwenken zumindest eines optischen Bauelements innerhalb eines endoskopischen Systems bekannt. Das Bauelement ist in den Strahlengang des endoskopischen Systems einschwenkbar und aus dem Strahlengang wieder ausschwenkbar, wobei die Schwenkachse schräg zur optischen Achse verlaufend angeordnet ist.

Gemäß EP 2 505 989 A1 weist eine Vorrichtung zur Fluoreszenzdiagnose ein Beleuchtungssystem zum Beleuchten eines Zielgebiets und ein Beobachtungssystem zum Beobachten des Zielgebiets in einem Weißlichtmodus und in einem Fluoreszenzmodus auf, wobei das Beobachtungssystem zumindest einen ersten Bildsensor zum Empfangen eines Weißlichtbildes und zumindest einen zweiten Bildsensor zum Empfangen eines Fluoreszenzbildes aufweist. Dem zweiten Bildsensor im Beobachtungspfad ist ein Beobachtungsspektralfilter zugeordnet, dessen Filtercharakteristik so ausgelegt ist, dass dem zweiten Bildsensor Licht im Spektralbereich zu beobachtender Fluoreszenz zugespeist werden kann, während Licht in den Spektralbereichen außerhalb der Fluoreszenz abgeblockt wird.

Das Beobachtungssystem kann ein Stereo-Endoskop aufweisen. Dabei weist das Stereo-Endoskop zwei Optikkanäle auf, wobei in jedem der beiden Optikkanäle ein Bildsensor angeordnet ist, von denen der eine zum Empfangen eines Fluoreszenzbildes dient, wobei diesem Bildsensor das Beobachtungsspektralfilter zugeordnet ist, das in den Strahlengang eingeschwenkt werden kann.

Aus EP 2 514 357 A1 ist eine Vorrichtung zum Beobachten und Beleuchten eines Objektfelds an einem Patienten von einer Stelle abseits vom Körper des Patienten bekannt, die ein Linsensystem zum Beobachten des Objektfelds in einer Blickrichtung und eine Beleuchtung zum Beleuchten des Objektfelds aufweist. Die Vorrichtung weist weiterhin einen Schaft auf, an dessen distalem Ende ein im Vergleich zu dem Durchmesser des Schafts erweitertes Kopfteil angeordnet ist, in dem zumindest eine ausstrahlende Beleuchtungseinheit zur homogenen Ausleuchtung des Objektfelds bei Abständen von einigen Zentimetern bis zu einem Meter angeordnet ist. Im Schaft sind Energieleitungen für die zumindest eine Beleuchtungseinheit angeordnet. Ferner ist im Schaft ein Stablinsensystem aufgenommen, das das Bild des Operationsfelds zu einem proximalen Ende des Schafts weiterleitet. Eine derartige Vorrichtung wird auch als "Exoskop" bezeichnet. Exoskope der genannten Art werden von der Fa. KARL STORZ GmbH & Co. KG unter der Bezeichnung VITOM ^{®} angeboten.

Die nicht vorveröffentlichte Europäische Patentanmeldung EP 2 850 996 A1 offenbart eine Vorrichtung zum Aufnehmen eines Bildes eines Objektfelds an einem menschlichen oder tierischen Körper von außerhalb des Körpers, die einen Schaft und eine an einem distalen Ende des Schafts angeordnete Beobachtungsoptik zum Aufnehmen des Bildes des Objektfelds umfasst, wobei die Beobachtungsoptik als Stereooptik ausgebildet ist. Hierdurch wird eine verbesserte räumliche Wahrnehmung des Objektfelds ermöglicht. Da sich bei einer Drehung des Schafts um eine näherungsweise parallel zur Blickrichtung der Optik stehende Achse nicht nur das auf einem elektronischen Bildaufnehmer erzeugte Bild des Operationsfelds, sondern zusätzlich auch die Basislinie der stereoskopischen Optik dreht, ist die Beobachtungsoptik dabei von einer Optikeinheit umfasst, die um eine zu einer Blickrichtung der Beobachtungsoptik näherungsweise parallele erste Drehachse drehbar ist. Hierdurch wird es ermöglicht, auch nach einem Schwenken der Vorrichtung das aufgenommene stereoskopische Bild mit einer im Wesentlichen ungeänderten Ausrichtung sowie in einer solchen Weise darzustellen, dass für einen Benutzer bzw. Beobachter der Stereoeffekt wahrnehmbar ist.

Es ist Aufgabe der vorliegenden Erfindung, eine gattungsgemäße Vorrichtung zu schaffen, die die Möglichkeit bietet, ein Bild des Objektfelds in unterschiedlichen Spektralbereichen aufzunehmen, insbesondere ein stereoskopisches Weißlichtbild und ein Fluoreszenzbild des Objektfelds aufzunehmen, wobei gleichzeitig ein kompakter Aufbau des distalen Endbereichs der Vorrichtung bzw. der drehbaren Optikeinheit ermöglicht werden soll.

Diese Aufgabe wird gelöst durch eine Vorrichtung zum Aufnehmen eines Bildes eines Objektfelds an einem menschlichen oder tierischen Körper von außerhalb des Körpers, umfassend einen Schaft und eine an einem distalen Ende des Schafts angeordnete Optikeinheit, die eine Beobachtungsoptik zum Aufnehmen des Bildes des Objektfelds umfasst und die um eine zu einer Blickrichtung der Beobachtungsoptik parallele Drehachse relativ zur Vorrichtung drehbar ist, wobei die Beobachtungsoptik einen ersten und einen zweiten Stereokanal mit jeweils einem Objektiv und jeweils mindestens einem elektronischen Bildaufnehmer aufweist, wobei die Beobachtungsoptik mindestens ein Filter umfasst, das in einen Strahlengang der Beobachtungsoptik einschwenkbar und aus diesem herausschwenkbar ist, wobei das mindestens eine Filter um eine parallel zur Drehachse gerichtete Schwenkachse schwenkbar ist. Vorteilhafte Weiterbildungen der Erfindung ergeben sich aus den Unteransprüchen.

Eine erfindungsgemäße Vorrichtung zum Aufnehmen eines Bilds eines Objektfelds an einem menschlichen oder tierischen Körper von einer Position außerhalb des menschlichen bzw. tierischen Körpers, die auch als "Exoskop" bezeichnet wird, umfasst einen Schaft, der vorzugsweise lang erstreckt und starr ausgebildet ist. Alternativ kann der Schaft auch sehr kurz ausgebildet sein. Das Objektfeld ist an einer Oberfläche des menschlichen oder tierischen Körpers angeordnet oder zumindest teilweise von außerhalb des Körpers beobachtbar, insbesondere ist das Objektfeld ein Operationsfeld einer chirurgischen Operation. Außerhalb bedeutet hier insbesondere, dass die Vorrichtung sich zu jedem Zeitpunkt, insbesondere auch beim Aufnehmen des Bilds des Objektfelds, vollständig außerhalb des Körpers befindet. Vorzugsweise hat die Vorrichtung dabei einen Abstand von mindestens ca. 15 cm, beispielsweise im Bereich von etwa 20 bis 75 cm, zum Körper und ist zum Aufnehmen des Bilds in einem entsprechenden Arbeitsabstand ausgebildet.

Die erfindungsgemäße Vorrichtung weist eine an einem distalen Ende des Schafts angeordnete Optikeinheit auf, die eine Beobachtungsoptik zum Aufnehmen des Bildes des Objektfelds umfasst und die um eine zu einer Blickrichtung der Beobachtungsoptik zumindest näherungsweise parallele Drehachse relativ zur Vorrichtung drehbar ist. Die Optikeinheit ist insbesondere in einem im Vergleich zu dem Durchmesser des Schafts erweiterten Kopfteil angeordnet. Die Beobachtungsoptik ist als Stereooptik ausgebildet und umfasst einen ersten und einen zweiten Stereokanal, die jeweils ein Objektiv und jeweils mindestens einen elektronischen Bildaufnehmer umfassen, wobei das Objektiv jeweils ein Bild des Objektfelds auf dem mindestens einen dem Objektiv zugeordneten elektronischen Bildaufnehmer erzeugt. Der mindestens eine elektronische Bildaufnehmer ist insbesondere ein halbleiterbasierter Bildsensor, beispielsweise ein CMOS- oder CCD-Sensor. Das erzeugte Bild kann jeweils ein Halbbild eines Stereobilds des Objektfelds darstellen. Die beiden aufgenommenen Halbbilder können einem Beobachter derart dargestellt werden, dass der Beobachter einen räumlichen Eindruck des Objektfelds gewinnt. Hierfür kann in an sich bekannter Weise beispielsweise ein Bildschirm mit einer wechselnden Polarisierung vorgesehen sein, wobei der Beobachter eine Polarisationsbrille mit unterschiedlichen Polarisierungen der beiden Gläser trägt.

Das Objektiv des ersten und das des zweiten Stereokanals sind voneinander in einer Richtung quer zur Blickrichtung beabstandet. Die Objektive und die Bildaufnehmer sind innerhalb der Optikeinheit angeordnet. Insbesondere sind die Objektive in einer festen räumlichen Beziehung zueinander angeordnet, wobei der quer zur Blickrichtung gemessene Abstand zwischen dem Objektiv des ersten Stereokanals und dem des zweiten Stereokanals die Stereobasis darstellt. Die Objektive sind mit ihren jeweiligen optischen Achsen parallel oder in einem Winkel zueinander angeordnet, der durch die Stereobasis und einem bevorzugten mittleren Arbeitsabstand, beispielsweise etwa 40 cm, bestimmt ist. Durch eine winklige Anordnung kann es erreicht werden, dass bei dem bevorzugten mittleren Arbeitsabstand die Mitte jedes Halbbilds des stereoskopischen Bilds denselben Punkt des Objektfelds darstellt. Als "Blickrichtung" der Beobachtungsoptik wird in dem Fall, dass die optischen Achsen der Objektive in einem Winkel zueinander stehen, eine zwischen den optischen Achsen stehende mittlere Richtung, insbesondere entsprechend einer Winkelhalbierenden der optischen Achsen, bezeichnet. Vorzugsweise ist die erste Drehachse parallel zur Blickrichtung der Beobachtungsoptik gerichtet oder nur um maximal einen solchen Winkel gegenüber der Blickrichtung geneigt, dass derjenige Punkt, in dem die Drehachse das Objektfeld durchstößt, in den von den elektronischen Bildaufnehmern des ersten und des zweiten Stereokanals aufgenommenen stereoskopischen Halbbildern unabhängig von der Drehung der Optikeinheit um die erste Drehachse enthalten ist. In besonders bevorzugter Weise fällt die Drehachse mit einer Mittellinie zwischen den optischen Achsen, beispielsweise mit der Winkelhalbierenden, zusammen.

Innerhalb des Schafts sind Versorgungs- und Signalleitungen zur Versorgung der elektronischen Bildaufnehmer und zur Weiterleitung der aufgenommenen Bilder, insbesondere der aufgenommenen Halbbilder des Stereobilds, zu einem proximalen Endbereich des Schafts aufgenommen, wo Anschlüsse zum Anschließen von Versorgungs-, Auswertungs- und/oder Anzeigeeinrichtungen vorgesehen sein können. Ferner können im Schaft beispielsweise Lichtleiter zum Weiterleiten von Beleuchtungslicht zum distalen Ende des Schafts angeordnet sein, wo eine Beleuchtungseinrichtung zum Beleuchten des Objektfelds vorgesehen sein kann. In bevorzugter Weise ist die Beleuchtungseinrichtung proximal der Beobachtungsoptik im distalen Endbereich der Vorrichtung, insbesondere am gegenüber dem Schaft erweiterten Kopfteil der Vorrichtung angeordnet. Das Exoskop kann durch eine Halterung gehalten werden, die einen gelenkigen Arm aufweist, an dessen distalem Ende ein Greifer angeordnet ist, der zum festen Halten des Exoskops, beispielsweise zum Greifen und Klemmen des Schafts, ausgebildet ist. Durch Verstellen der Halterung kann das Exoskop je nach den gestellten Anforderungen in einem geeigneten Abstand zum Objektfeld und in einer geeigneten Position und Ausrichtung fixiert werden. Wenn die Optik des Exoskops als Seitblickoptik ausgebildet ist mit einer Blickrichtung, die mit der Längsachse des Schafts einen Winkel von etwa von 90° bildet, kann das Exoskop zum Beobachten eines horizontal angeordneten Objektfelds, das etwa ein Operationsfeld bei einer chirurgischen Operation am menschlichen Körper sein kann, insbesondere über dem Objektfeld mit senkrecht nach unten gerichteter Blickrichtung positioniert werden. Durch Verstellen der Halterung kann das Exoskop dann, je nach Bedarf und je nachdem von welcher Seite ein ungehinderter Zugang zum Operationsfeld erforderlich ist, bei näherungsweise gleichbleibender Blickrichtung in unterschiedliche Positionen gebracht und hierfür um eine vertikale Achse geschwenkt werden.

Erfindungsgemäß umfasst die Beobachtungsoptik mindestens ein optisches Filter, das insbesondere als Transmissions-Filter ausgebildet ist, das in einen Strahlengang der Beobachtungsoptik, d.h. in einen Strahlengang mindestens eines der Stereokanäle, einschwenkbar und aus diesem wieder herausschwenkbar ist. Nicht erfindungsgemäß ist das mindestens eine Filter um eine im Wesentlichen senkrecht zur Drehachse gerichtete Schwenkachse schwenkbar. Insbesondere ist das mindestens eine Filter um eine im Wesentlichen senkrecht zur Blickrichtung gerichtete Schwenkachse schwenkbar. Das mindestens eine Filter kann zum Einschwenken in den Strahlengang und zum Ausschwenken aus dem Strahlengang innerhalb der Optikeinheit entsprechend schwenkbar gelagert sein, wobei durch die Lagerung die Schwenkachse definiert wird. Die Schwenkachse verläuft vorzugsweise durch die Optikeinheit und ist weiter vorzugsweise in einer unveränderlichen Lage relativ zur Optikeinheit angeordnet. Als "Einschwenken" wird im vorliegenden Zusammenhang eine Schwenk- oder Rotationsbewegung des mindestens einen Filters bezeichnet, durch die das Filter derart positioniert wird, dass alle oder zumindest ein für die Bilderzeugung wesentlicher Anteil der Strahlen des betreffenden Strahlengangs durch das Filter hindurchtreten; als "Ausschwenken" wird dementsprechend eine Schwenk- oder Rotationsbewegung des Filters bezeichnet, wodurch das Filter derart angeordnet wird, dass alle oder zumindest ein für die Bilderzeugung wesentlicher Anteil der Strahlen des betreffenden Strahlengangs nicht durch das Filter hindurchtreten, bevor sie auf den betreffenden Bildaufnehmer gelangen. Insbesondere ist eine jeweilige Endposition der Bewegung, die als "Einschwenken" bzw. "Ausschwenken" bezeichnet wird, derart gewählt, dass die auf den elektronischen Bildaufnehmer des betreffenden Stereokanals auftreffende Strahlung durch das mindestens eine Filter gefiltert bzw. nicht gefiltert wird; in diesem Fall ist das mindestens eine Filter somit zwischen einer ersten Endposition, in der das Filter innerhalb des Strahlengangs der Beobachtungsoptik angeordnet ist und einer zweiten Position, in der es außerhalb des Strahlengangs angeordnet ist, schwenkbar. Die Optikeinheit kann weitere mechanische, optische und/oder elektronische Bauelemente umfassen.

Dadurch, dass die Beobachtungsoptik mindestens ein Filter aufweist, das um eine im Wesentlichen nicht erfindungsgemäß senkrecht zur Drehachse der Optikeinheit stehende Schwenkachse schwenkbar und dadurch in einen Strahlengang mindestens eines der Stereokanäle einbringbar und aus diesem wieder entfernbar ist, kann auf einfache Weise die wahlweise Aufnahme von Bildern des Objektfelds in unterschiedlichen Spektralbereichen ermöglicht werden, wobei zugleich eine raumsparende Anordnung des mindestens einen Filters innerhalb der drehbaren Optikeinheit ermöglicht wird. Insbesondere wird hierdurch eine kompakte Bauweise der drehbaren Optikeinheit ermöglicht, so dass ein ebenfalls raumsparender Aufbau des distalen Endbereichs bzw. des Kopfteils der erfindungsgemäßen Vorrichtung ermöglicht wird. Insbesondere kann die Optikeinheit als kompakte Einheit mit einer im Wesentlichen zylindrischen Mantelfläche ausgebildet werden. Hierdurch kann ein Stereo-Exoskop geschaffen werden, das eine stereoskopische Beobachtung eines Objektfelds ermöglicht, etwa eines Operationsfelds bei einer offenen chirurgischen Operation am menschlichen Körper, ohne dass ein Zugang des Operateurs zum Operationsfeld wesentlich eingeschränkt wird, und das zudem die wahlweise Beobachtung des Objektfelds in unterschiedlichen Spektralbereichen ermöglicht.

Das mindestens eine Filter ist vorzugsweise in Form einer planparallelen Platte ausgebildet, wobei die Schwenkachse insbesondere in einer zur Oberfläche des Filters im Wesentlichen parallelen Ebene liegen kann. Weiter vorzugsweise ist in der in den Strahlengang des ersten und/oder des zweiten Stereokanals eingeschwenkten Position die Normale auf der Oberfläche des plattenförmigen Filters im Wesentlichen in Richtung der optischen Achse des Strahlengangs des betreffenden Stereokanals und insbesondere im Wesentlichen parallel zur Drehachse gerichtet, während sie in der ausgeschwenkten Position schräg oder näherungsweise senkrecht zur Drehachse gerichtet ist. Hierdurch werden eine Verringerung von Reflexionsverlusten und eine verbesserte Filterwirkung des Filters sowie eine besonders raumsparende Anordnung des Filters ermöglicht.

Gemäß einer bevorzugten Ausführungsform der Erfindung liegen die optischen Achsen der Objektive des ersten und des zweiten Stereokanals in einer gemeinsamen Ebene. In dieser Ebene liegt in dem Fall, dass die optischen Achsen der Objektive in einem Winkel zueinander stehen, auch die zwischen den optischen Achsen stehende mittlere Richtung, insbesondere die Winkelhalbierende, die die Blickrichtung definiert. Vorzugsweise liegt die Schwenkachse des mindestens einen Filters zumindest näherungsweise in der gemeinsamen Ebene oder in einer zu dieser parallelen Ebene, wobei im letzteren Fall die Schwenkachse in vorteilhafter Weise durch die Optikeinheit verläuft, insbesondere nahe an der Drehachse vorbei, oder wobei die Schwenkachse die Drehachse schneidet. Hierdurch wird eine weiter verbesserte Raumausnutzung bei der schwenkbaren Anordnung des mindestens einen Filters innerhalb der Optikeinheit ermöglicht.

Vorzugsweise umfasst die Beobachtungsoptik ein erstes und ein zweites Filter, wobei das erste Filter in den Strahlengang des ersten Stereokanals und das zweite Filter in den Strahlengang des zweiten Stereokanals einschwenkbar und aus diesem wieder ausschwenkbar ist. Hierdurch wird ein besonders einfacher Aufbau einer Stereooptik mit in die jeweiligen Strahlengänge einbringbaren Filtern geschaffen. Gemäß einer alternativen bevorzugten Ausführungsform kann es vorgesehen sein, dass ein einziges schwenkbares Filter zwei Filterbereiche umfasst, von denen ein erster Filterbereich in den Strahlengang des ersten Stereokanals und ein zweiter Filterbereich in den Strahlengang des zweiten Stereokanals einschwenkbar und aus diesem wieder ausschwenkbar ist. Auch hierdurch ist ein besonders einfacher Aufbau erreichbar.

In besonders bevorzugter Weise ist das erste Filter in den Strahlengang des ersten Stereokanals zwischen das Objektiv und den mindestens einen Bildaufnehmer des ersten Stereokanals einschwenkbar, und das zweite Filter ist in den Strahlengang des zweiten Stereokanals zwischen das Objektiv und den Bildaufnehmer des zweiten Stereokanals einschwenkbar; dies kann entsprechend für den ersten und den zweiten Filterbereich gemäß der zuvor erwähnten alternativen Ausführungsform gelten. Dadurch, dass das jeweilige Filter bzw. der jeweilige Filterbereich zwischen Objektiv und Bildaufnehmer einbringbar ist, kann ein besonders kompakter Aufbau der Optikeinheit erreicht werden.

Vorzugsweise sind das erste und das zweite Filter auf einem gemeinsamen, um die Schwenkachse schwenkbaren Filterträger angeordnet. Der Filterträger kann insbesondere U-förmig ausgebildet sein, wobei die beiden äußeren Schenkel beispielsweise Lagerzapfen tragen, durch die die Schwenkachse verläuft, und wobei der mittlere Schenkel mit einer ebenen Fläche ausgebildet ist, die zwei Fenster aufweist, in die das erste und das zweite Filter eingesetzt sind, und durch die in der eingeschwenkten Position die Strahlengänge der Stereokanäle hindurchtreten. In dem Fall, dass ein durchgehendes Filter mit einem ersten und einem zweiten Filterbereich gemäß der zuvor erwähnten alternativen Ausführungsform vorgesehen ist, kann dieses in ein zum Einschwenken in die Strahlengänge ausgebildetes Fenster eines im Übrigen gleichartig ausgebildeten Filterträgers eingesetzt sein. Auf diese Weise wird eine weiter vereinfachte und besonders raumsparende Anordnung ermöglicht, bei der durch die Ausgestaltung des Filterträgers eine weiter verbesserte Ausnutzung des innerhalb der Optikeinheit vorhandenen Raums möglich ist und ferner auf einfache Weise erreicht werden kann, dass das erste und das zweite Filter gleichzeitig in den Strahlengang des jeweiligen Stereokanals eingebracht und aus diesem wieder entfernt werden können.

Gemäß einer bevorzugten Ausführungsform der Erfindung weisen das erste und das zweite Filter eine im Wesentlichen gleiche spektrale Charakteristik auf, insbesondere im Wesentlichen gleiche spektrale Transmissionsfunktionen. Die spektrale Charakteristik der Filter kann insbesondere zur Beobachtung von Fluoreszenzstrahlung in einem gleichen Fluoreszenzmodus ausgebildet sein. So können beispielsweise das erste und das zweite Filter beide zur Beobachtung von mittels Protoporphyrin IX erzeugter Fluoreszenz oder beide zur Beobachtung von Autofluoreszenzstrahlung ausgebildet sein. Hierfür können das erste und das zweite Filter wie in der Europäischen Patentanmeldung EP 2 505 989 A1 beschrieben ausgebildet sein.

Wenn das erste und das zweite Filter in die Strahlengänge des ersten bzw. des zweiten Stereokanals eingeschwenkt sind, ist die Vorrichtung somit zur stereoskopischen Beobachtung des Objektfelds in entsprechender Fluoreszenzstrahlung eingerichtet; sind die Filter ausgeschwenkt, kann ein Stereobild des Objektfelds beispielsweise im Weißlicht aufgenommen werden. Anstelle eines ersten und eines zweiten Filters kann ein einziges Filter mit einem entsprechenden ersten und einem zweiten Filterbereich vorgesehen sein.

Alternativ können das erste und das zweite Filter unterschiedliche spektrale Charakteristiken aufweisen, insbesondere unterschiedliche spektrale Transmissionsfunktionen. So kann beispielsweise das erste Filter zur Beobachtung von induzierter Fluoreszenz, etwa durch Protoporphyrin IX induzierter Fluoreszenz, und das zweite Filter zur Beobachtung von Autofluoreszenz des Gewebes ausgebildet sein. Hierfür können das erste und das zweite Filter die zuvor erwähnten, jedoch unterschiedlichen spektralen Charakteristiken aufweisen. Hierdurch wird es ermöglicht, mit einer einfach aufgebauten Anordnung unterschiedliche Fluoreszenzmodi zu beobachten sowie ein stereoskopisches Bild des Objektfelds im Weißlicht aufzunehmen.

Gemäß einer bevorzugten Ausführungsform der Erfindung ist das mindestens eine Filter motorisch schwenkbar, d.h. zum Einschwenken in den Strahlengang bzw. zum Ausschwenken aus dem Strahlengang der Beobachtungsoptik motorisch antreibbar. Insbesondere umfasst die Optikeinheit eine motorische Antriebseinrichtung, die einen Elektromotor und ein Getriebe umfassen kann oder beispielsweise mit einem Hubmagneten oder als Tauchspulenantrieb ausgebildet sein kann. Hierdurch wird es insbesondere ermöglicht, das mindestens eine Filter zum Einbringen in den Strahlengang bzw. zum Entfernen aus dem Strahlengang von außerhalb der Optikeinheit anzusteuern.

Vorzugsweise umfasst die Optikeinheit mindestens einen Sensor zur Erfassung einer Schwenkposition des mindestens einen Filters. Hierdurch wird es ermöglicht zu erkennen, in welcher Position sich das mindestens eine Filter befindet bzw. das Ein- und Ausschwenken des Filters zu überwachen.

Der mindestens eine Sensor kann in vorteilhafter Weise als magnetischer Sensor ausgebildet sein, wobei beispielsweise ein Magnet mit dem mindestens einen schwenkbaren Filter bzw. mit dem schwenkbaren Filterträger verbunden und der magnetische Sensor, der zur Erfassung des Magnetfelds des Magneten dient, an einer Grundstruktur der drehbaren Optikeinheit angeordnet sein kann; umgekehrt kann beispielsweise der Sensor mit dem mindestens einen schwenkbaren Filter bzw. mit dem schwenkbaren Filterträger verbunden und der Magnet an der Grundstruktur der Optikeinheit befestigt sein. Hierdurch wird auf einfache Weise eine Überwachung der Schwenkbewegung des mindestens einen Filters ermöglicht.

Der mindestens eine Sensor kann insbesondere als Näherungsschalter ausgebildet sein, der zur Überwachung des Erreichens einer Endposition der Schwenkbewegung des mindestens einen Filters angeordnet und ausgebildet ist. Ein solcher Näherungsschalter kann beispielsweise wie zuvor beschrieben als magnetischer Sensor oder in anderer Weise, etwa mittels elektrischen Kontakten oder als kapazitiver Näherungssensor, ausgebildet sein. Da für eine ausreichende Filterung bzw. einen ungefilterten Durchgang der zu den elektronischen Bildaufnehmern gelangenden Strahlung insbesondere das Erreichen der Endpositionen der Schwenkbewegung des mindestens einen Filters wesentlich sein kann, ist hierdurch eine Überwachung der Schwenkbewegung auf besonders einfache Weise möglich.

Gemäß einer bevorzugten Ausführungsform der Erfindung umfasst die Beobachtungsoptik eine Fokussiereinrichtung. Die Fokussiereinrichtung umfasst insbesondere eine im Wesentlichen parallel zur optischen Achse des Objektivs des ersten und/oder zweiten Stereokanals gerichtete Führung, entlang derer das betreffende Objektiv motorisch verschiebbar ist. Vorzugsweise sind das Objektiv des ersten und das des zweiten Stereokanals an einem gemeinsamen Schlitten angeordnet, der an mindestens einer Führung im Wesentlichen in Blickrichtung der Beobachtungsoptik verschiebbar ist. Hierdurch wird es ermöglicht, die Beobachtungsoptik an unterschiedliche Arbeitsabstände heranzupassen. Ferner wird eine Nachfokussierung ermöglicht, die aufgrund des Ein- oder Ausschwenkens des mindestens einen Filters in den bzw. aus dem Strahlengang der Beobachtungsoptik und der dadurch verursachten Veränderung der optischen Weglänge notwendig sein kann. In besonders bevorzugter Weise sind die Objektive motorisch verschiebbar, wodurch eine automatische Nachfokussierung und/oder eine Ansteuerung der Fokussierung von außerhalb der Optikeinheit ermöglicht wird.

Vorzugsweise umfasst die Optikeinheit eine Markierungslichtquelle, die einen im Wesentlichen parallel zur Blickrichtung der Beobachtungsoptik gerichteten Markierungslichtstrahl erzeugt. Hierfür kann die Optikeinheit beispielsweise einen Laser oder eine lichtemittierende Diode (LED) sowie eine entsprechende Kollimatoroptik aufweisen. Die Markierungslichtquelle ermöglicht die Markierung des Objektfelds bzw. eines Teilbereichs des Objektfelds. Vorzugsweise erzeugt die Markierungslichtquelle Markierungslicht mit einer Wellenlänge im sichtbaren Spektralbereich, jedoch außerhalb eines mit der Beobachtungsoptik beobachtbaren Fluoreszenzbereichs, so dass ein aufgenommenes Fluoreszenzbild durch das Markierungslicht nicht beeinträchtigt wird. Hierdurch kann für einen Benutzer der Vorrichtung ein Bereich, in dem ein Bild, insbesondere ein Fluoreszenzbild, des Objektfelds aufgenommen wird, leicht erkennbar gemacht werden.

Gemäß einer besonders bevorzugten Ausführungsform der Erfindung umfassen der erste und der zweite Stereokanal jeweils mindestens einen weiteren elektronischen Bildaufnehmer, insbesondere genau einen weiteren elektronischen Bildaufnehmer, sowie einen Strahlteiler, wobei der Strahlteiler zur Aufteilung der in den jeweiligen Stereokanal eingetretenen Strahlung auf die mindestens zwei Bildaufnehmer des jeweiligen Stereokanals angeordnet und ausgebildet ist. In besonders bevorzugter Weise sind die Strahlteiler des ersten und des zweiten Stereokanals jeweils dichroitisch strahlteilend ausgebildet, so dass auf die mindestens zwei Bildaufnehmer des jeweiligen Stereokanals Strahlungsanteile in unterschiedlichen Wellenlängenbereichen gelangt. So kann beispielsweise der Strahlteiler derart ausgebildet sein, dass sichtbares Licht auf einen ersten elektronischen Bildaufnehmer reflektiert wird, während infrarotes Licht in Transmission zu dem weiteren elektronischen Bildaufnehmer gelangt. Hierdurch kann es insbesondere ermöglicht werden, durch Indocyaningrün (ICG) induzierte Fluoreszenzstrahlung mit dem weiteren Bildaufnehmer zu erfassen, während ein Bild im sichtbaren Spektralbereich mit dem ersten Bildaufnehmer aufgenommen wird.

Vorzugsweise ist die drehbare Optikeinheit hermetisch dicht ausgebildet. Die Optikeinheit kann hierfür ein hermetisch dichtes Gehäuse umfassen, das auf der dem Objektfeld zugewandten Seite ein oder mehrere, hermetisch dicht in das Gehäuse eingesetzte Deckgläser aufweist. Insbesondere ist das Gehäuse derart hermetisch dicht ausgebildet, dass auch unter den bei der Sterilisation in einem Autoklaven auftretenden Temperatur- und Druckbedingungen kein Eindringen von Dampf in das Gehäuse möglich ist oder zumindest nicht in einem wesentlichen Umfang erfolgen kann. Das Gehäuse bildet somit eine Hülle, durch die die Optikeinheit hermetisch von der Umgebung getrennt ist. Vorzugsweise ist das Gehäuse metallisch, insbesondere aus einem nicht-ferromagnetischen Metall, ausgebildet; hierfür sind beispielsweise Aluminium und austenitische Stähle geeignet. Innerhalb des hermetisch dichten Gehäuses sind insbesondere das Objektiv und der mindestens eine elektronische Bildaufnehmer des ersten und des zweiten Stereokanals und das mindestens eine schwenkbare Filter sowie ggf. ein schwenkbarer Filterträger, eine Antriebseinrichtung des schwenkbaren Filters, eine Fokussiereinrichtung und/oder Strahlteiler und weitere elektronische Bildaufnehmer aufgenommen. Elektrische Versorgungs- und Signalleitungen können durch das Gehäuse abgedichtet geführt sein, um die Bildaufnehmer sowie ggf. die Antriebe des Filters und/oder der Fokussiereinrichtung anzusteuern. Zusätzlich oder alternativ zur hermetisch dichten Ausbildung der Optikeinheit kann ein Gehäuse der Vorrichtung, insbesondere eines Kopfteils der Vorrichtung, in dem die drehbare Optikeinheit aufgenommen ist, und/oder ein Innengehäuse der Vorrichtung, das die optischen Bauteile aufnimmt, oder auch die gesamte Vorrichtung hermetisch dicht ausgebildet sein. Diesbezüglich kann die Vorrichtung wie in den Europäischen Patentanmeldungen EP 14185795.3 und/oder EP 14185857.1 ausgebildet sein Hierdurch wird es ermöglicht, auf einfache Weise die hygienischen Anforderungen bei einem Einsatz im sterilen Bereich eines Operationssaals zu erfüllen, ohne dass die Lebensdauer der in der Optikeinheit enthaltenen optischen und elektronischen Bauelemente wesentlich eingeschränkt würde.

Zur manuellen Drehung der Optikeinheit zur Aufrichtung des aufgenommenen Bilds und zur Einstellung der Stereobasis kann ein Griffrad, etwa eine Drehkappe, vorgesehen sein, das bzw. die mit der drehbar gelagerten Optikeinheit wirkverbunden ist. Zusätzlich oder stattdessen kann ein elektromotorischer Antrieb zur Drehung der Optikeinheit vorgesehen sein. Ein solcher Antrieb zur manuellen oder elektromotorischen Drehung der Optikeinheit kann beispielsweise gemäß der Europäischen Patentanmeldung EP 14185795.3 ausgebildet sein. Ein motorischer Antrieb zur Drehung der Optikeinheit kann aber auch etwa einen in der Optikeinheit angeordneten Elektromotor umfassen, der ein Zahnrad antreibt, das exzentrisch zur Drehachse in der Optikeinheit gelagert ist und das über eine Außenkontur der Optikeinheit hinausragt. Das Zahnrad greift in einen innerhalb des Kopfteils der Vorrichtung angeordneten, konzentrisch zur Drehachse ausgebildeten Zahnkranz ein, so dass durch Ansteuern des Elektromotors eine Drehung der Optikeinheit um die Drehachse bewirkt werden kann. Ein motorischer Antrieb der Optikeinheit kann eine besonders einfache Ausrichtung und insbesondere in dem Fall, dass eine Schwenkbewegung der Vorrichtung durch einen oder mehrere Sensoren erfasst wird, eine automatische Ausrichtung der Stereooptik ermöglichen.

In vorteilhafter Weise ist die Optikeinheit wärmeleitend mit einem im Schaft verlaufenden Wärmeleiter verbunden. Hierdurch wird die Abführung der beim Betrieb der elektronischen Bildaufnehmer sowie ggf. weiterer elektrischer Verbraucher, etwa der Antriebe und/oder der Markierungslichtquelle, entstehenden Verlustwärme ermöglicht. Hierfür kann es beispielsweise vorgesehen sein, dass eine im Schaft verlaufende Heatpipe mit ihrem distalen Ende mit einer Lagerung der Optikeinheit wärmeleitend verbunden ist oder direkt an einer Außenseite der Optikeinheit anliegt. Insbesondere kann die Optikeinheit auf wärmeleitenden Gleitlagern, beispielsweise aus Bronze oder Graphitfolie, gelagert sein. Alternativ oder zusätzlich kann ein Abstreifer aus einem metallischen Material oder auch aus Graphit an einer Außenfläche der Optikeinheit zur Wärmeabführung anliegen. Die auf diese Weise in den Schaft übertragene Wärme kann durch die Heatpipe zum proximalen Ende des Schafts abführbar sein, wo sie in den Griff weitergeleitet und über die Oberfläche des Griffs abgegeben werden kann; im proximalen Endbereich des Schafts oder im Griff kann aber auch ein Wärmetauscher zur weiteren Abführung der Wärme in eine externe Kühleinrichtung angeordnet sein. Durch derartige Maßnahmen zur Wärmeabführung kann auch die Abführung von durch eine Beleuchtungseinrichtung in den Schaft und dessen distalen Endbereich eingebrachter Verlustwärme ermöglicht werden.

Vorzugsweise ist mindestens ein Kabel vorgesehen zur Versorgung, Steuerung und/oder Signalübertragung der elektronischen Bildaufnehmer und ggf. weiterer elektrischer und elektronischer Baugruppen, die innerhalb der drehbaren Optikeinheit angeordnet sind. Insbesondere kann das Kabel innerhalb des Schafts der Vorrichtung geführt sein und von einem proximalen Endabschnitt des Schafts bis zu einem distalen Endabschnitt des Schafts bzw. zu einem am distalen Ende des Schafts angeordneten Kopfteil verlaufen. Am proximalen Endabschnitt des Schafts oder auch an einem mit dem proximalen Endabschnitt des Schafts verbundenen Handgriff können Anschlüsse zur Vermittlung einer Verbindung zu externen Versorgungs-, Auswertungs- und/oder Anzeigeeinrichtungen vorgesehen sein. Das Kabel kann beispielsweise als Flachbandkabel oder Flexplatine ausgebildet sein und auf einem zylindrischen Bereich der Außenseite der Optikeinheit aufwickelbar sein. Der distale Endbereich des Exoskops, insbesondere das gegenüber dem Schaft erweiterte Kopfteil, kann einen inneren Hohlraum aufweisen, in dem ein Kabelreservoir aufgenommen ist, das einen bei einer Drehung der Optikeinheit aufgewickelten Kabelabschnitt bereitstellt und einen bei einer Drehung der Optikeinheit in einer umgekehrten Drehrichtung abgewickelten Kabelabschnitt aufnimmt. Insbesondere können das Kabel, die Kabelführung und das Kabelreservoir wie in der Europäischen Patentanmeldung EP 14185857.1 beschrieben ausgebildet sein.

In besonders bevorzugter Weise umfasst die Vorrichtung eine Beleuchtungsoptik zum Beleuchten des Objektfelds. Die Beleuchtungsoptik kann wie in der Europäischen Patentanmeldung EP 2 514 357 A1 beschrieben ausgebildet sein.

Die Beleuchtungsoptik des Exoskops kann eine Lichtquelle zur Erzeugung von Beleuchtungslicht umfassen oder an eine externe Lichtquelle anschließbar sein. Die Lichtquelle ist vorzugsweise zum wahlweisen Erzeugen von Beleuchtungslicht unterschiedlicher spektraler Zusammensetzung ausgebildet, insbesondere zum Erzeugen von Beleuchtungslicht, das für eine Beobachtung des Objektfelds im Weißlicht und/oder in einem oder mehreren Fluoreszenzmodi geeignet ist. In vorteilhafter Weise kann die Beleuchtungseinrichtung bzw. die Lichtquelle synchron mit einer Antriebseinrichtung des mindestens einen Filters ansteuerbar sein, um zur Beobachtung mindestens eines Fluoreszenzmodus ein entsprechendes Ein- oder Ausschwenken des Filters synchron mit der Erzeugung einer hierfür geeigneten Fluoreszenzanregungsstrahlung zu steuern. Hierdurch wird eine einfache Beobachtung des Objektfelds in unterschiedlichen Spektralbereichen, insbesondere in einem oder mehreren Fluoreszenzmodi, ermöglicht.

Gemäß einer Ausführungsform der Erfindung umfasst die Beobachtungsoptik mindestens ein optisches Filter, das insbesondere als Transmissions-Filter ausgebildet ist, das in einen Strahlengang der Beobachtungsoptik, d.h. in einen Strahlengang mindestens eines der Stereokanäle, einschwenkbar und aus diesem in einen seitlich zu den Strahlengängen beider Stereokanäle angeordneten Raumbereich wieder herausschwenkbar ist. Eine derart ausgebildete gattungsgemäße Vorrichtung ist somit als Stereo-Exoskop mit einem ersten und einem zweiten Stereokanal ausgebildet, wobei die beiden Strahlengänge in der Optikeinheit zueinander benachbart angeordnet sind, wobei insbesondere die optischen Achsen der jeweiligen Objektive in einer gemeinsamen Ebene verlaufen und wobei seitlich neben den beiden Strahlengängen ein Raumbereich vorgesehen ist, in dem das mindestens eine Filter angeordnet ist, wenn es aus dem Strahlengang herausgeschwenkt ist. Dieser Raumbereich ist insbesondere in einer Richtung quer zu einer gemeinsamen Ebene der optischen Achsen der Objektive angeordnet und erstreckt sich in einer senkrechten Projektion auf die gemeinsame Ebene über eine im Wesentlichen gleiche Ausdehnung wie die Strahlengänge der Stereokanäle. In besonders bevorzugter Weise erstreckt sich der Raumbereich innerhalb der drehbaren Optikeinheit, beispielsweise innerhalb eines zylindrischen Gehäuses, das die Strahlengänge der Stereokanäle raumsparend umschließt.

Gemäß der Erfindung ist erkannt worden, dass es in vorteilhafter Weise möglich ist, den durch die Nebeneinanderanordnung der Strahlengänge der beiden Stereokanäle einer Stereooptik entstehenden Raum neben den Strahlengängen der Stereokanäle für die Aufnahme des mindestens einen schwenkbaren Filters zu nutzen, wenn dieses aus dem Strahlengang der Beobachtungsoptik ausgeschwenkt ist. Hierdurch wird ein raumsparender Aufbau des distalen Endbereichs bzw. des Kopfteils der erfindungsgemäßen Vorrichtung ermöglicht, wobei die Optikeinheit insbesondere als kompakte Einheit mit einer im Wesentlichen zylindrischen Mantelfläche ausgebildet werden kann.

Die Vorrichtung bzw. die drehbare Optikeinheit gemäß der Erfindung kann beispielsweise wie oben beschrieben ausgebildet sein, wobei dadurch, dass das mindestens eine Filter um eine im Wesentlichen senkrecht zur Drehachse der Optikeinheit gerichtete Schwenkachse schwenkbar ist, erreichbar sein kann, dass das Filter in seiner herausgeschwenkten Position in einem seitlich zu den Strahlengängen beider Stereokanäle angeordneten Raumbereich angeordnet ist.

Erfindungsgemäß ist das mindestens eine Filter um eine im Wesentlichen parallel zur Drehachse gerichtete Schwenkachse schwenkbar, wobei hierdurch das Filter aus dem Strahlengang mindestens eines der Stereokanäle in einen seitlich zu den Strahlengängen beider Stereokanäle angeordneten Raumbereich herausschwenkbar ist. Insbesondere kann hierfür eine Schwenkachse des mindestens einen Filters in einem Bereich zwischen den Strahlengängen der Stereokanäle oder derart angeordnet sein, dass der Abstand der Schwenkachse von der Drehachse geringer ist als der Abstand der optischen Achsen der Strahlengänge der Stereokanäle von der Drehachse, die mit einer Mittellinie zwischen den beiden optischen Achsen zusammen fallen kann. Auch hierdurch wird eine raumsparende Anordnung des schwenkbaren Filters und somit ein kompakter Aufbau der Optikeinheit, insbesondere mit einer im Wesentlichen zylindrischen Mantelfläche, ermöglicht.

Insbesondere kann die Optikeinheit ein erstes Filter und zweites Filter umfassen, wobei das erste Filter in einen Strahlengang des ersten Stereokanals und das zweite Filter in einen Strahlengang des zweiten Stereokanals einschwenkbar und aus diesem ausschwenkbar ist, und wobei das erste Filter in axialer Richtung, bezogen auf die Drehachse, gegenüber dem zweiten Filter versetzt ist. Weiterhin vorzugsweise kann das erste Filter um eine erste, im Wesentlichen parallel zur Drehachse gerichtete Schwenkachse und das zweite Filter um eine zweite, der ersten benachbarte und ebenfalls im Wesentlichen parallel zur Drehachse gerichtete Schwenkachse schwenkbar sein. Hierdurch kann eine besonders raumsparende Anordnung erreichbar sein. Im Übrigen kann die Vorrichtung bzw. die Optikeinheit wie oben beschrieben ausgebildet sein.

Gemäß einem erfindungsgemäßen Verfahren zum Aufnehmen eines Bildes eines Objektfelds an einem menschlichen oder tierischen Körper von außerhalb des Körpers wird eine Vorrichtung, die wie oben beschrieben ausgebildet ist, in einer Beobachtungsposition außerhalb des Körpers gehalten, beispielsweise mittels eines Greifers an einem gelenkigen, verstellbaren Arm einer entsprechenden Halterung. In der Beobachtungsposition ist das Objektfeld in einem Arbeitsabstand in der Blickrichtung der Vorrichtung angeordnet, insbesondere in einem Abstand von mindestens ca. 15 cm zur Vorrichtung, beispielsweise in einem Abstand im Bereich von etwa 20 bis 75 cm. Die Vorrichtung befindet sich zu jedem Zeitpunkt, insbesondere auch beim Aufnehmen des Bilds des Objektfelds, vollständig außerhalb des Körpers. Gemäß dem erfindungsgemäßen Verfahren kann die Optikeinheit mit der Beobachtungsoptik zur Ausrichtung einer Stereobasis der Stereooptik manuell und/oder motorisch um die Drehachse der Optikeinheit gedreht werden. Weiter wird gemäß dem erfindungsgemäßen Verfahren mindestens ein in der Optikeinheit aufgenommenes Filter in einen Strahlengang der Beobachtungsoptik, d.h. mindestens eines Stereokanals, eingeschwenkt und mit dem mindestens einen elektronischen Bildaufnehmer, nämlich dem Bildaufnehmer des mindestens einen Stereokanals, ein Bild des Objektfelds in einem ersten Spektralbereich aufgenommen, der insbesondere durch die Transmissionsfunktion des eingeschwenkten Filters und durch eine erste spektrale Zusammensetzung des Beleuchtungslichts bestimmt ist. Sofern ein oder mehrere entsprechende Filter in den ersten und den zweiten Stereokanal eingeschwenkt werden, kann das aufgenommene Bild ein Stereobild in dem ersten Spektralbereich sein. Weiter wird das mindestens eine Filter aus dem Strahlengang der Beobachtungsoptik in einen seitlich zu den Strahlengängen beider Stereokanäle angeordneten Raumbereich innerhalb der Optikeinheit ausgeschwenkt und mit dem jeweils mindestens einen elektronischen Bildaufnehmer des ersten und des zweiten Stereokanals ein Stereobild des Objektfelds in einem zweiten Spektralbereich aufgenommen, der insbesondere durch eine zweite spektrale Zusammensetzung des Beleuchtungslichts bestimmt ist. Die erste spektrale Zusammensetzung des Beleuchtungslichts kann zur Anregung einer Fluoreszenzstrahlung geeignet sein, die mittels des mindestens einen eingeschwenkten Filters beobachtet wird, und die zweite spektrale Zusammensetzung kann beispielsweise Weißlicht sein. Dadurch, dass das mindestens eine Filter in einen seitlich zu den Strahlengängen beider Stereokanäle angeordneten Raumbereich innerhalb der Optikeinheit ausgeschwenkt wird, kann eine kompakte Ausgestaltung der drehbaren Optikeinheit und somit eine Ausgestaltung des distalen Endbereichs der Vorrichtung ermöglicht werden, die den Zugang zum Objektfeld nicht wesentlich einschränkt. Insbesondere kann die Schwenkachse, um die das mindestens eine Filter ein- und ausgeschwenkt wird, im Wesentlichen senkrecht zur Drehachse der Optikeinheit gerichtet sein oder ist erfindungsgemäß im Wesentlichen parallel zur Drehachse der Optikeinheit.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung zweier bevorzugter Ausführungsbeispiele und der beigefügten Zeichnung. Es zeigen:
Fig. 1a und 1b ein Ausführungsbeispiel einer erfindungsgemäßen Vorrichtung, wobei Fig. 1b eine teiltransparente Darstellung ist;
Fig. 2 eine Optikeinheit gemäß einem nicht erfinderischen Ausführungsbeispiel;
Fig. 3 einen Stereokanal der Optikeinheit gemäß Fig. 2 in einem Längsschnitt;
Fig. 4 ausgewählte optische Elemente der Optikeinheit gemäß Fig. 2 in vereinfachter Darstellung;
Fig. 5a bis 5c eine Fokussiereinrichtung einer Optikeinheit gemäß Fig. 2 in drei unterschiedlichen Ansichten;
Fig. 6 eine Optikeinheit gemäß Fig. 2 entgegen der Blickrichtung gesehen;
Fig. 7 ausgewählte optische Elemente einer Optikeinheit gemäß der Erfindung in vereinfachter Darstellung.

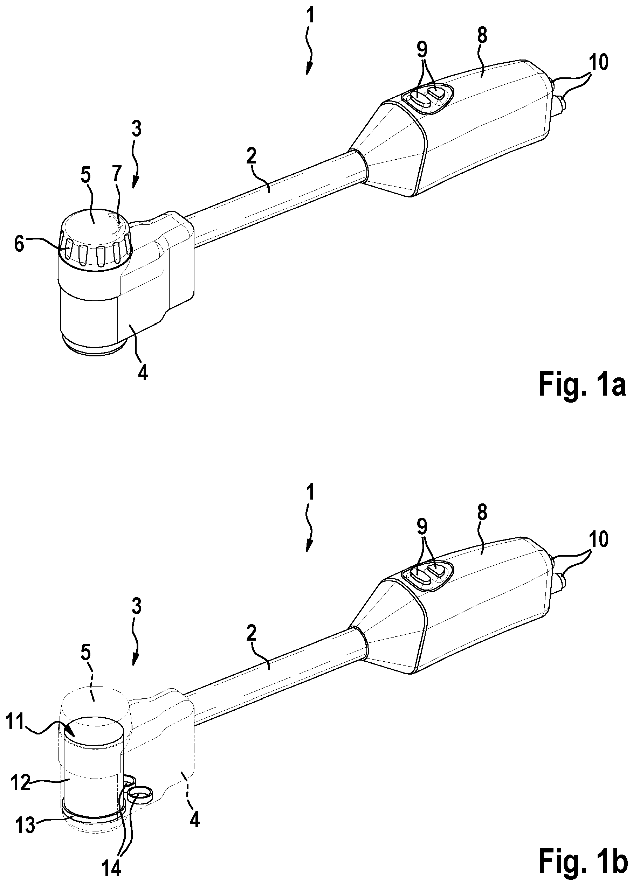

Eine erfindungsgemäße Vorrichtung, die im Folgenden als Exoskop 1 bezeichnet wird, ist in den Figuren 1a und 1b in einer perspektivischen Ansicht gezeigt. Das Exoskop 1 weist einen zylindrischen Schaft 2 auf, an dessen distalem Ende ein im Vergleich zum Schaft 2 erweitertes Kopfteil 3 angeordnet ist. Das Kopfteil 3 weist ein Außengehäuse 4 auf, dessen distaler Bereich näherungsweise halbzylindrisch geformt ist, wobei die Achse des Halbzylinders zur Längsachse des Schafts 2 in einem Winkel von etwa 90° steht. Auf den näherungsweise halbzylindrischen distalen Endbereich des Außengehäuses 4 ist eine Drehkappe 5 aufgesetzt, die an ihrer Umfangsseite eine Mehrzahl von Griffmulden 6 aufweist. Ferner weist die Drehkappe 5 an ihrer Oberseite eine Markierung 7 auf, die die Drehstellung der Drehkappe 5 leicht erkennbar macht. Wie durch die Pfeile der Markierung 7 angedeutet, ist die Drehkappe 5 manuell drehbar, um eine im Inneren des Außengehäuses 4 angeordnete drehbare Optikeinheit um eine zur Längsachse des Schafts 2 senkrecht stehende Drehachse zu drehen. Die drehbare Optikeinheit 11 ist in der Darstellung der Figur 1b, in der das Außengehäuse 4 und die Drehkappe 5 transparent dargestellt sind, erkennbar. Die Optikeinheit 11 ist im Wesentlichen zylindrisch ausgebildet, wobei die Achse des Zylinders mit der Drehachse, um die die Optikeinheit 11 mittels der Drehkappe 5 gedreht werden kann, zusammenfällt. Ferner sind in Fig. 1b an der Unterseite des Außengehäuses 4 angeordnete Beleuchtungsfenster 14 angedeutet, durch die Beleuchtungslicht in Richtung auf ein zu beobachtendes Objektfeld abgestrahlt wird. Weiter weist das Exoskop 1 einen Handgriff 8 auf, der an dem dem Kopfteil 3 gegenüberliegenden, proximalen Ende des Schafts 2 angeordnet ist und der Bedienelemente 9 sowie Anschlüsse 10 für elektrische Signal- und Versorgungsleitungen sowie für ein Lichtleitkabel zur Zuführung von Beleuchtungslicht trägt.

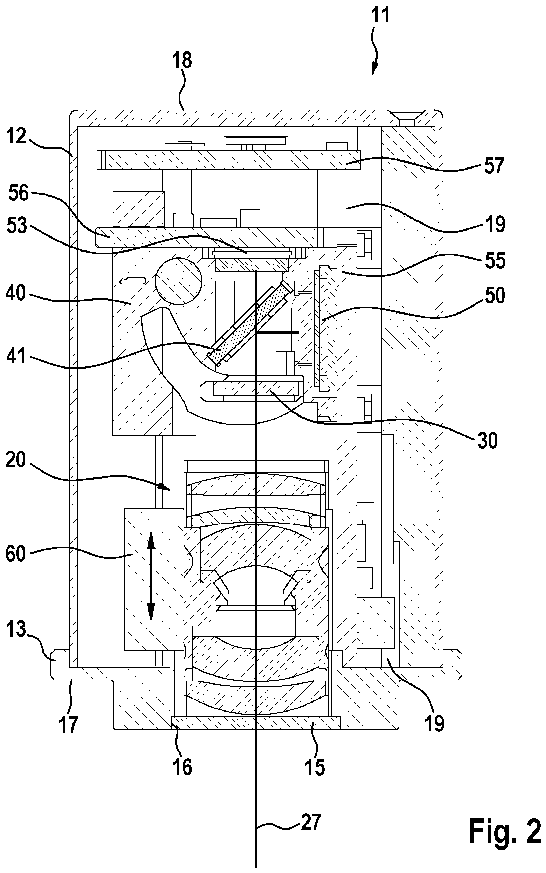

In Fig. 2 ist die drehbare Optikeinheit 11 in einem schematischen Längsschnitt gezeigt. Die Optikeinheit 11 kann hermetisch dicht ausgebildet sein, wofür ein im Wesentlichen zylindrisches Gehäuseoberteil 12 dicht mit der Grundplatte 13 verbunden und ein in eine Ausnehmung 16 der Grundplatte 13 eingesetztes Deckglas 15 hermetisch dicht eingesetzt sein kann. Alternativ oder zusätzlich kann das Außengehäuse 4 bzw. das Kopfteil 3 des Exoskops 1 oder das Exoskop 1 insgesamt, d.h. einschließlich des Schafts 2 und des Handgriffs 8 (s. Fig. 1a und 1b), hermetisch dicht ausgebildet sein. Das Deckglas 15 kann auch als optisches Filter ausgebildet sein oder, insbesondere in dem Fall, dass zumindest das Außengehäuse 4 hermetisch dicht ist, entfallen. Ein stufenförmiger Absatz 17 der Grundplatte 13 dient als Lagerfläche der drehbaren Optikeinheit 11 im Kopfteil 4 (s. Fig. 1a, 1b), ebenso wie die Oberseite 18 des Gehäuseoberteils 12. Der Absatz 17, die Oberseite 18 sowie die übrigen Bereiche der Außenseiten des Gehäuseoberteils 12 und der Grundplatte 13 dienen zur Abführung von innerhalb der Optikeinheit 11 entstehender Verlustwärme.

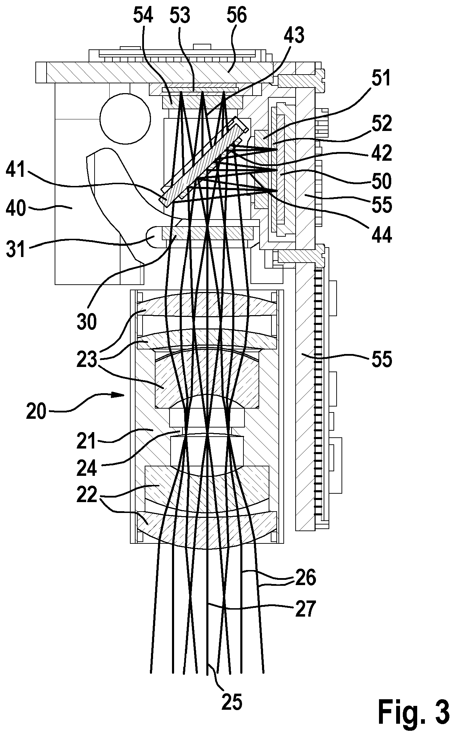

Innerhalb des durch das Gehäuseoberteil 12, die Grundplatte 13 und das Deckglas 15 gebildeten Gehäuses der Optikeinheit 11 ist die Beobachtungsoptik des Exoskops 1 angeordnet, von der in Fig. 2 einer der beiden Stereokanäle dargestellt ist. Die einzelnen Teilsysteme der Beobachtungsoptik sind in den Figuren 3 bis 5c im Einzelnen dargestellt und werden im Folgenden mit Bezug auf die genannten Figuren beschrieben.

Der in den Figuren 2 und 3 dargestellte Stereokanal umfasst ein Objektiv 20, das ein Objektivgehäuse 21 aufweist, das als Fassung der optischen Bauelemente des Objektivs 20 dient, insbesondere der Linsen 22, 23, und weiterhin eine Objektivblende 24 trägt. Das Objektiv 20 ist im Wesentlichen zylindrisch ausgebildet, wobei die Zylinderachse mit der optischen Achse 25 des Objektivs 20 zusammenfällt. Bei der Beobachtung eines Objektfelds mit dem Exoskop 1 ist die optische Achse 25 zum Objektfeld gerichtet. In Fig. 3 ist der Strahlengang durch das Objektiv 20 anhand einiger vom Objektfeld einfallender Strahlen 26 dargestellt; in Fig. 2 ist der Strahlengang lediglich symbolisch durch einen Zentralstrahl 27, der mit der optischen Achse 25 zusammenfällt, angedeutet.

In den Figuren 2 und 3 ist gezeigt, dass in den Strahlengang des dargestellten Stereokanals nach dem Objektiv 20 ein optisches Filter 30 eingeschwenkt ist, das insbesondere ein Transmissionsfilter sein kann, dessen spektrale Charakteristik für die Beobachtung von Fluoreszenzstrahlung angepasst ist. Das Filter 30 ist als planparallele Platte ausgebildet, die in der in Fig. 2 und 3 gezeigten Stellung senkrecht zur optischen Achse 25 des Objektivs 20 steht. Das Filter 30 ist in einem Filterträger 31 gehalten, der in einem Träger 40 der Optikeinheit 11 um eine zur optischen Achse 25 nicht erfindungsgemäß senkrechte, in den Figuren 2 und 3 senkrecht zur Zeichenebene stehende Schwenkachse schwenkbar gelagert ist.

Im Träger 40 ist weiterhin ein Strahlteiler 41 unter einem Winkel von etwa 45° zur optischen Achse 25 des Objektivs 20 gehalten. Der Strahlteiler 41 ist als planparallele Platte mit einer dichroitisch strahlteilenden Beschichtung 42 ausgebildet, so dass vom Objektfeld kommende Strahlen 26 nach Durchlaufen des Objektivs 20 und des Filters 30 in einen transmittierten Strahl 43 und einen reflektierten Strahl 44, welche spektral unterschiedlich zusammengesetzt sind, aufgeteilt werden. Die dichroitisch strahlteilenden Eigenschaften der Beschichtung 42 sind dabei derart gewählt, dass Strahlungsanteile im sichtbaren Spektralbereich in den reflektierten Strahl 44 gelangen, während infrarote Spektralanteile den transmittierten Strahl 43 bilden. Die reflektierten Strahlen 44 treffen auf einen Bildsensor 50, beispielsweise einen CMOS- oder einen CCD-Sensor, der als elektronischer Bildaufnehmer zum Aufnehmen eines Bilds des Objektfelds im sichtbaren Spektralbereich dient. Die Sensorfläche des Bildsensors 50 ist in einer Bildebene des Objektivs 20 angeordnet. Insbesondere ist der Bildsensor 50 im gesamten sichtbaren Spektralbereich empfindlich und nimmt daher in den Fall, dass das Objektfeld mit einer breitbandigen Beleuchtungsstrahlung beleuchtet wird und das Filter 30 aus dem Strahlengang herausgeschwenkt ist, ein Weißlichtbild des Objektfelds auf. Um zu vermeiden, dass das vom Bildsensor 50 aufgenommene Weißlichtbild durch etwaige in den reflektierten Strahlengang gelangende restliche Infrarotanteile gestört wird, ist vor dem Bildsensor 50 ein Infrarotblockfilter 51 angeordnet, das am Träger 40 beispielsweise durch Kleben befestigt ist. Zum Schutz des Bildsensors 50 ist vor diesem eine Abdeckplatte 52 vorgesehen. Im transmittierten Strahlengang ist ein weiterer elektronischer Bildaufnehmer, nämlich ein Bildsensor 53, mit seiner Sensorfläche in einer Bildebene des Objektivs 20 angeordnet, um ein Bild des Objektfelds im infraroten Spektralbereich aufzunehmen. Der weitere elektronische Bildaufnehmer bzw. der weitere Bildsensor 53 kann ebenfalls ein CMOS- oder ein CCD-Sensor sein. Vor dem weiteren Bildsensor 53 kann ein ICG-Filter 54 angeordnet sein, dessen spektrale Transmission für die Beobachtung ICG-induzierter Fluoreszenz ausgelegt ist.

Der Bildsensor 50 und der weitere Bildsensor 53 sind jeweils auf Platinen 55, 56 angeordnet, die elektronische Bauelemente und Schaltkreise zur Versorgung und Steuerung der Bildsensoren 50, 53 sowie zur Signalverarbeitung und -übertragung enthalten. Wie in Fig. 2 gezeigt, können eine oder mehrere weitere Platinen 57 mit weiteren elektronischen Bauelementen in der Optikeinheit 11 vorgesehen sein.

Die spektrale Charakteristik der in der Optikeinheit 11 sowie in einer Lichtquelle, die das Beleuchtungslicht zur Beleuchtung des Objektfelds erzeugt, verwendeten Filter kann beispielsweise so gewählt sein wie in der Europäischen Patentanmeldung EP 2 505 989 A1 oder in der Europäischen Patentanmeldung EP 2 609 849 A1 beschrieben ist. Insbesondere können die Filter 30, 30' für die Beobachtung von durch Protoporphyrin IX induzierter Fluoreszenz eine Transmission im Wellenlängenbereich zwischen 380 und 430 nm von weniger als 0,1% und zwischen 450 und 800 nm von mehr als 96% aufweisen, sowie für die Beobachtung von Autofluoreszenz im Bereich von 380 bis 455 nm von weniger als 0,1 % und im Bereich von 475 bis 800 nm von mehr als 96 %. In der Lichtquelle können Filter zur Erzeugung einer entsprechend angepassten Fluoreszenzanregungsstrahlung vorgesehen sein. Vereinfacht ausgedrückt, liegt bei der Beobachtung von Pp IX-Fluoreszenz die Beleuchtungsstrahlung zur Anregung des Fluoreszenzfarbstoffes im Bereich von ca. 405 nm, und die Beobachtung der Fluoreszenzstrahlung erfolgt im Bereich von ca. 630 nm, während bei der Beobachtung der Autofluoreszenz die Anregung in einem breiten Spektralbereich im blauen Bereich und die Beobachtung breitbandig bei Wellenlängen oberhalb von ca. 450 nm erfolgt. Die ICG-Fluoreszenz kann mit Beleuchtungsstrahlung von ca. 785 nm angeregt und bei Wellenlängen von ca. 800 bis 900 nm beobachtet werden; das ICG-Filter 54 ist hierfür in dem letztgenannten Wellenlängenbereich und vorzugsweise nur in diesem durchlässig. Die Beleuchtungsstrahlung für die Weißlicht-Beobachtung, die bei ausgeschwenkten Filtern 30, 30' erfolgt und gleichzeitig mit der Beobachtung der ICG-Fluoreszenz erfolgen kann, kann den gesamten sichtbaren Spektralbereich umfassen.

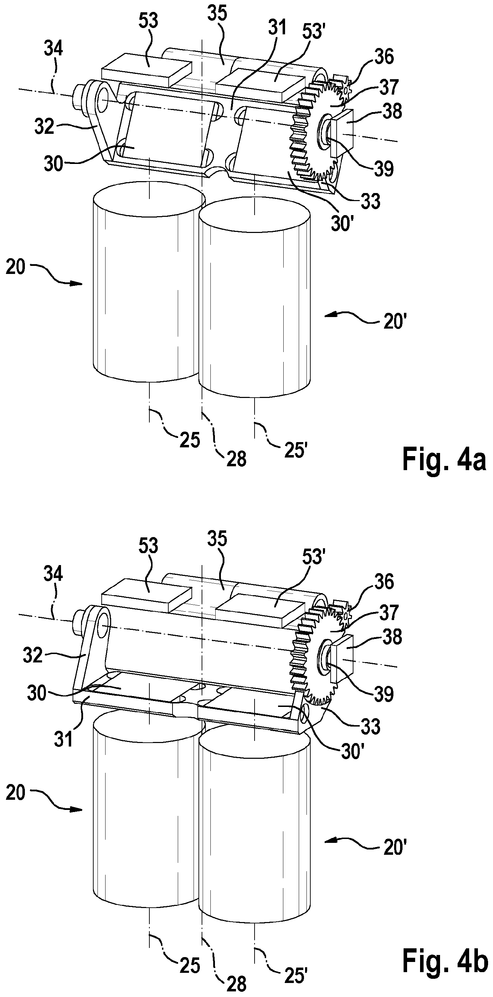

In Fig. 4a und 4b ist in schematischer Form gezeigt, dass für jeden der beiden Stereokanäle der Optikeinheit 11 ein einschwenkbares Filter 30, 30' vorgesehen ist. Wie in Fig. 4a und 4b in vereinfachter Form dargestellt, umfasst der erste Stereokanal ein Objektiv 20 und einen Bildsensor 53, wobei der Einfachheit halber nur ein einziger Bildsensor gezeigt und der Strahlteiler 41 nicht dargestellt ist; es können jedoch, wie in Fig. 2 und 3 gezeigt, mehrere Bildsensoren 50, 53 und ein Strahlteiler 41 zur Aufteilung der Beobachtungsstrahlung auf die Bildsensoren 50, 53 vorhanden sein. Ein zweiter Stereokanal wird durch das Objektiv 20' und den Bildsensor 53' gebildet, wobei im zweiten Stereokanal ebenfalls zwei Bildsensoren und ein Strahlteiler vorhanden sein können. Die Bildsensoren 53, 53' sind jeweils in einer Bildebene des Objektivs 20, 20' angeordnet.

Die Drehachse 28 der Optikeinheit 11 ist in der Mitte zwischen den optischen Achsen 25, 25' der beiden Objektive 20, 20' angeordnet. Die Blickrichtung fällt mit der Drehachse 28 zusammen und ist zum Objektfeld, d.h. in der Darstellung der Figuren 4a und 4b nach unten, gerichtet. In den Figuren 4a und 4b verlaufen die optischen Achsen 25, 25' der Objektive 20, 20' parallel zueinander; die Drehachse 28 ist daher parallel zu den beiden optischen Achsen 25, 25'. Die optischen Achsen 25, 25' der Objektive 20, 20' können aber auch beispielsweise in einem solchen Winkel zueinander stehen, dass die optischen Achsen 25, 25' sich im Bereich eines bevorzugten Arbeitsabstands schneiden; in diesem Fall ist die Drehachse 28 insbesondere die Winkelhalbierende zwischen den beiden optischen Achsen 25, 25'.

In den Strahlengang des ersten und des zweiten Stereokanals zwischen das Objektiv 20, 20' und den jeweils zugeordneten Bildsensor 53, 53' ist jeweils ein Filter 30, 30' einschwenkbar. Die beiden Filter 30, 30' sind in einem gemeinsamen Filterträger 31 gehalten. Der Filterträger 31 ist insgesamt etwa U-förmig ausgebildet und mit den Enden der Schenkel 32, 33 in dem nicht dargestellten Träger 40 der Optikeinheit 11 (s. Fig. 2, 3) schwenkbar gelagert. Nicht erfindungsgemäß verläuft die Schwenkachse 34 senkrecht zu den optischen Achsen 25, 25' der Objektive 20, 20' und liegt in einer durch die optischen Achsen 25, 25' gebildeten Ebene. Die Schwenkachse 34 kann die Drehachse 28 schneiden oder nahe an dieser vorbeilaufen. Dadurch, dass der Filterträger 31 um die Schwenkachse 34 schwenkbar ist, können die Filter 30, 30' aus den Strahlengängen der Stereokanäle ausgeschwenkt und in diese zwischen dem Objektiv 20, 20' und dem jeweils zugeordneten Bildsensor 53, 53' eingeschwenkt werden.

Zum Durchführen der Schwenkbewegung ist der Filterträger 31 mit einer Antriebseinrichtung verbunden, die einen Elektromotor 35 umfasst sowie ein erstes Zahnrad 36, das auf der Motorwelle des Elektromotors 35 befestigt ist, und ein mit dem ersten Zahnrad 36 kämmendes zweites Zahnrad 37, das mit dem Filterträger 31 bzw. mit dem Schenkel 33 verbunden ist. Der Elektromotor 35 kann beispielsweise ein Schrittmotor sein. Durch Ansteuerung des Elektromotors 35 kann eine Schwenkbewegung des Filterträgers 31 in einer gewünschten Richtung durchgeführt werden. Zur Erfassung einer Schwenkposition des Filterträgers 31 ist ein Magnetsensor 38 vorgesehen, der mit einem mit dem Zahnrad 37 verbundenen Magneten 39 zusammenwirkt.

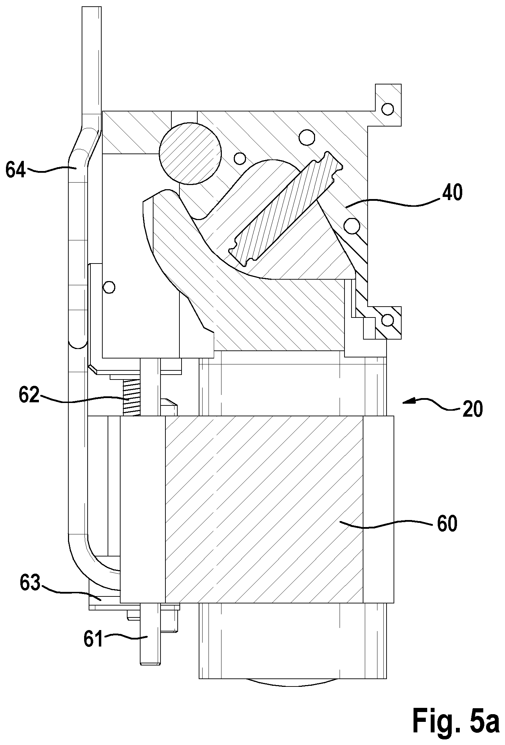

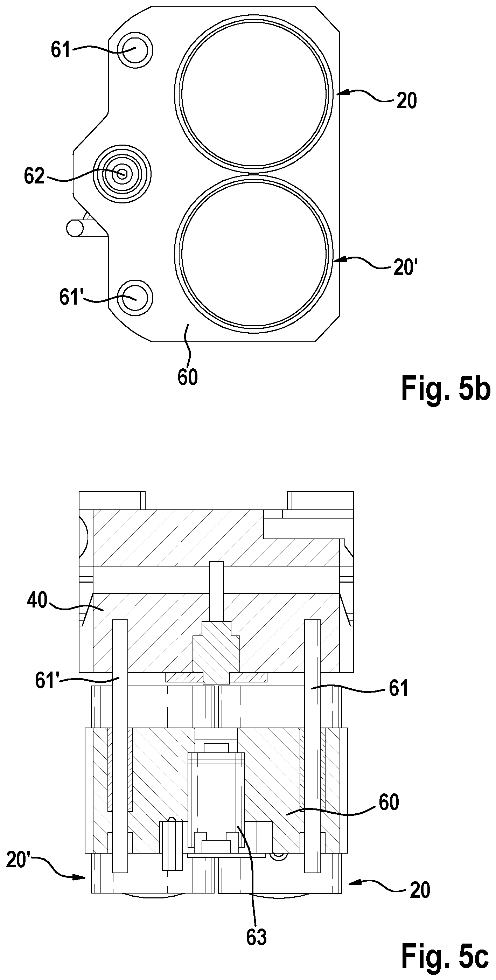

In den Figuren 5a bis 5c ist eine Fokussiereinrichtung dargestellt, die zum Nachfokussieren beim Ein- bzw. Ausschwenken der Filter 30, 30' aus dem jeweiligen Strahlengang sowie zur Anpassung an unterschiedliche Arbeitsabstände zum beobachteten Objektfeld dient. In Fig. 5a sind in einer den Figuren 2 und 3 entsprechenden Ansicht das Objektiv 20 sowie der Träger 40 der Optikeinheit 11 dargestellt; die optischen Bauelemente sind hier der Deutlichkeit halber nicht gezeigt. Wie in Fig. 5a zu erkennen ist, ist das Objektiv 20 in einem in axialer Richtung verschiebbaren Schlitten 60 aufgenommen, der an einer Gleitführung 61 geführt und mit einer Gewindespindel 62 verschiebbar ist. Die Gleitführung 61 und die Gewindespindel 62 sind am Träger 40 befestigt. Mit dem Schlitten 60 ist ein Schrittmotor 63 verbunden, der eine nicht dargestellte Spindelmutter der Gewindespindel 62 antreibt, um den Schlitten 60 entlang der Gleitführung 61 zu verschieben. Ferner ist in Fig. 5a ein Kabel 64 schematisch dargestellt, das der Versorgung des Schrittmotors 63 dient. Wie in der in Fig. 5b gezeigten, aus axialer Richtung vom Objektfeld her gesehenen Ansicht erkennbar ist, wird der Schlitten 60 von zwei Gleitführungen 61, 61' geführt. Der Schlitten 60 ist mit Gleitbuchsen beispielsweise aus Messing, Kunststoff oder einem anderen geeigneten Material auf den Gleitführungen 61, 61' verschiebbar. Dadurch, dass zwei Gleitführungen 61, 61' mit jeweils einer langen Gleitbuchse oder zwei axial voneinander beabstandeten kürzeren Gleitbuchsen vorgesehen sind, ist der Schlitten 60 quer zur axialen Richtung statisch überbestimmt gelagert; hierdurch können eine Verschiebung der Objektive 20, 20' in einer Querrichtung sowie eine unerwünschte Drehung der Objektive 20, 20' relativ zum Träger 40 vermieden werden. Die Spindelmutter ist quer zur axialen Richtung mit einer Spielpassung am Schlitten 60 gelagert.

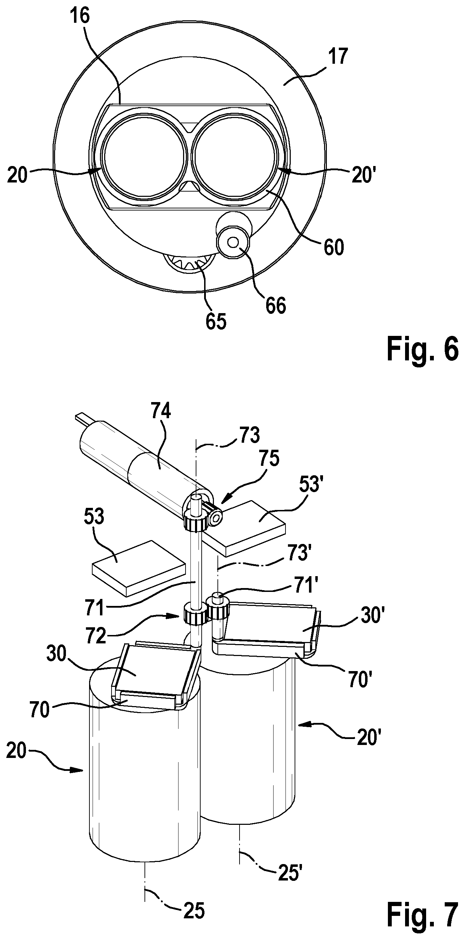

In Fig. 6 ist die drehbare Optikeinheit in einer Ansicht gezeigt, die der Ansicht der Fig. 5b entspricht. Wie in Fig. 6 zu erkennen ist, sind die Objektive 20, 20' der beiden Stereokanäle nebeneinander im Schlitten 60 aufgenommen, der durch die Ausnehmung 16 der Grundplatte 13 sichtbar ist. In die Ausnehmung 16 ist ein nicht dargestelltes Deckglas 15 eingesetzt. Ferner ist der als Lagerfläche und zur Wärmeabführung dienende Absatz 17 der Grundplatte 13 sowie ein auf einer Motorwelle eines in der Optikeinheit aufgenommenen, in Fig. 6 nicht sichtbaren Antriebsmotors befestigtes Zahnrad 65 gezeigt, das mit einem nicht dargestellten, fest mit dem Außengehäuse 4 des Exoskops 1 verbundenen innenverzahnten Zahnkranz kämmt und zum Drehen der Optikeinheit 11 um ihre Drehachse 28 dient (s. auch Fig. 1a, 1b, 4a, 4b). Ferner ist ein als Markierungslichtquelle dienender Laserpointer 66 dargestellt.

Fig. 7 zeigt ausgewählte optische Elemente der Beobachtungsoptik gemäß einer erfinderischen Ausführungsform der Erfindung in einer der Fig. 4b entsprechenden Darstellung. Zwischen das Objektiv 20, 20' und den Bildsensor 53, 53' der beiden Stereokanäle ist jeweils ein Filter 30, 30' eingeschwenkt. Zur Ausführung der Schwenkbewegung sind die Filter 30, 30' jeweils in einem Filterträger 70, 70' aufgenommen, der jeweils an einer Filterwelle 71, 71' befestigt ist. Die Filterwelle 71' des Filterträgers 70' des zweiten Stereokanals wird über ein in Fig. 7 symbolisch dargestelltes Getriebe 72 von der Filterwelle 71 des Filterträgers 70 des ersten Stereokanals angetrieben. Die Filterwelle 71 wird von einem Elektromotor 74 über ein ebenfalls symbolisch dargestelltes Getriebe 75 angetrieben. Die Filterwellen 71, 71' definieren eine jeweilige Schwenkachse 73, 73' des Filters 30, 30'. Die Schwenkachsen 73, 73' sind parallel zu den optischen Achsen 25, 25' der Objektive 20, 20' gerichtet und verlaufen zwischen den Objektiven 20, 20' oder in einem der Drehachse, die einer Mittellinie zwischen den optischen Achsen 25, 25' entspricht, nahe benachbarten Bereich; wie in Fig. 7 gezeigt, kann beispielsweise die Schwenkachse 73 mit der Drehachse, die in Fig. 7 entsprechend den Figuren 4a und 4b verläuft, identisch sein. Hierdurch wird es erreicht, dass die Filter 30, 30' aus der in Fig. 7 gezeigten, in die Strahlengänge der Stereokanäle eingeschwenkten Position in eine ausgeschwenkte Position geschwenkt werden können, die neben den Strahlengängen der Stereokanäle angeordnet ist, d.h. in der Darstellung der Fig. 7 in einem Raumbereich vor den Objektiven 20, 20' bzw. nach oben versetzt. Durch eine axial versetzte Anordnung der Filterträger 70, 70' kann erreicht werden, dass beide Filterträger 70, 70' auf dieselbe Seite der Strahlengänge geschwenkt werden können, d.h. in den Raumbereich vor den Objektiven 20, 20'. Im Übrigen kann das in Fig. 7 dargestellte Ausführungsbeispiel wie zu den Figuren 1 bis 6 beschrieben ausgebildet sein.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Exoskop
- 2: Schaft
- 3: Kopfteil
- 4: Außengehäuse
- 5: Drehkappe
- 6: Griffmulde
- 7: Markierung
- 8: Handgriff
- 9: Bedienelement
- 10: Anschluss
- 11: Optikeinheit
- 12: Gehäuseoberteil
- 13: Grundplatte
- 14: Beleuchtungsfenster
- 15: Deckglas
- 16: Ausnehmung
- 17: Absatz
- 18: Oberseite
- 19: Kühlkörper
- 20, 20': Objektiv
- 21: Objektivgehäuse
- 22: Linse
- 23: Linse
- 24: Objektivblende
- 25, 25': Optische Achse
- 26: Strahl
- 27: Zentralstrahl
- 28: Drehachse
- 30, 30': Filter
- 31: Filterträger
- 32: Schenkel
- 33: Schenkel
- 34: Schwenkachse
- 35: Elektromotor
- 36: Zahnrad
- 37: Zahnrad
- 38: Magnetsensor
- 39: Magnet
- 40: Träger
- 41: Strahlteiler
- 42: Beschichtung
- 43: Transmittierter Strahl
- 44: Reflektierter Strahl
- 50: Bildsensor
- 51: Infrarot-Blockfilter
- 52: Abdeckplatte
- 53, 53': Bildsensor
- 54: ICG-Filter
- 55: Platine
- 56: Platine
- 57: Platine
- 60: Schlitten
- 61, 61': Gleitführung
- 62: Gewindespindel
- 63: Schrittmotor
- 64: Kabel
- 65: Zahnrad
- 66: Laserpointer
- 70, 70': Filterträger
- 71, 71': Filterwelle
- 72: Getriebe
- 73, 73': Schwenkachse
- 74: Elektromotor
- 75: Getriebe

## Patentansprüche

1. Vorrichtung zum Aufnehmen eines Bildes eines Objektfelds an einem menschlichen oder tierischen Körper von außerhalb des Körpers, umfassend einen Schaft (2) und eine an einem distalen Ende des Schafts (2) angeordnete Optikeinheit (11), die eine Beobachtungsoptik zum Aufnehmen des Bildes des Objektfelds umfasst, wobei die Beobachtungsoptik einen ersten und einen zweiten Stereokanal mit jeweils einem Objektiv (20, 20') und jeweils mindestens einem elektronischen Bildaufnehmer (53, 53') aufweist, und die Optikeinheit (11) um eine zu einer Blickrichtung der Beobachtungsoptik parallele Drehachse (28) relativ zur Vorrichtung drehbar ist, wobei die Beobachtungsoptik mindestens ein Filter (30, 30') umfasst, das in einen Strahlengang der Beobachtungsoptik einschwenkbar und aus diesem herausschwenkbar ist, und das mindestens eine Filter (30, 30') um eine parallel zur Drehachse (28) gerichtete Schwenkachse (73, 73') schwenkbar ist.

2. Vorrichtung nach Anspruch 1, **dadurch gekennzeichnet, dass** die Beobachtungsoptik das erste Filter (30) und ein zweites Filter (30') umfasst, wobei das erste Filter (30) in einen Strahlengang des ersten Stereokanals und das zweite Filter (30') in einen Strahlengang des zweiten Stereokanals einschwenkbar und aus diesem ausschwenkbar ist.

3. Vorrichtung nach Anspruch 2, **dadurch gekennzeichnet, dass** das mindestens eine Filter (30, 30') aus dem Strahlengang in einen seitlich zu den Strahlengängen beider Stereokanäle angeordneten Raumbereich herausschwenkbar ist.

4. Vorrichtung nach Anspruch 2 oder 3, **dadurch gekennzeichnet, dass** das erste Filter (30) zwischen das Objektiv (20) und den mindestens einen Bildaufnehmer (53,53') des ersten Stereokanals und das zweite Filter (30') zwischen das Objektiv (20') und den mindestens einen Bildaufnehmer (53, 53') des zweiten Stereokanals einschwenkbar und aus diesem ausschwenkbar ist.

5. Vorrichtung nach Anspruch 3 oder 4, **dadurch gekennzeichnet, dass** der Raumbereich in einer Richtung quer zu einer gemeinsamen Ebene von optischen Achsen der Objektive (20, 20') angeordnet ist und sich in einer senkrechten Projektion auf die gemeinsame Ebene über eine gleiche Ausdehnung wie die Strahlengänge der Stereokanäle erstreckt.

6. Vorrichtung nach einem der Ansprüche 3 bis 5, **dadurch gekennzeichnet, dass** sich der Raumbereich innerhalb eines zylindrischen Gehäuses der drehbaren Optikeinheit erstreckt, welches die Strahlengänge der Stereokanäle raumsparend umschließt.

7. Vorrichtung nach Anspruch 5 oder 6, **dadurch gekennzeichnet, dass** die Schwenkachse (73, 73') des mindestens einen Filters (30, 30') in einem Bereich zwischen den Strahlengängen der Stereokanäle oder derart angeordnet ist, dass ein Abstand der Schwenkachse (73, 73') von der Drehachse (28) geringer ist als ein Abstand der optischen Achsen (25, 25') der Strahlengänge der Stereokanäle von der Drehachse (28).

8. Vorrichtung nach einem der Ansprüche 2 bis 7, **dadurch gekennzeichnet, dass** das erste Filter (30) in axialer Richtung, bezogen auf die Drehachse (28), gegenüber dem zweiten Filter (30') versetzt ist.

9. Vorrichtung nach einem der Ansprüche 2 bis 8, **dadurch gekennzeichnet, dass** das erste Filter (30) um eine erste, im Wesentlichen parallel zur Drehachse (28) gerichtete Schwenkachse (71) und das zweite Filter (30') um eine zweite, der ersten benachbarte und ebenfalls im Wesentlichen parallel zur Drehachse (28) gerichtete Schwenkachse (71') schwenkbar sind.

10. Vorrichtung nach einem der Ansprüche 2 bis 8, **dadurch gekennzeichnet, dass** das erste und das zweite Filter (30, 30') auf einem gemeinsamen schwenkbaren Filterträger angeordnet sind.

11. Vorrichtung nach einem der Ansprüche 2 bis 10, **dadurch gekennzeichnet, dass** das erste und das zweite Filter (30, 30') gleiche spektrale Charakteristiken oder unterschiedliche spektrale Charakteristiken aufweisen.

12. Vorrichtung nach einem der vorhergehenden Ansprüche, **dadurch gekennzeichnet, dass** die Optikeinheit (11) mindestens einen Sensor, insbesondere ausgebildet als Magnetsensor (38) und/oder als Näherungsschalter, zur Erfassung einer Schwenkposition des mindestens einen Filters (30, 30') umfasst.

13. Vorrichtung nach einem der vorhergehenden Ansprüche, **dadurch gekennzeichnet, dass** die Beobachtungsoptik eine Fokussiereinrichtung und/oder die Optikeinheit eine Markierungslichtquelle umfasst.

14. Vorrichtung nach einem der vorhergehenden Ansprüche, **dadurch gekennzeichnet, dass** der erste und der zweite Stereokanal jeweils mindestens einen weiteren elektronischen Bildaufnehmer (53, 53') sowie einen Strahlteiler (41) umfasst.

15. Vorrichtung nach einem der vorhergehenden Ansprüche, **dadurch gekennzeichnet, dass** die Optikeinheit (11) hermetisch dicht ausgebildet ist.

## Claims

1. Device for capturing an image of an object field on a human or animal body from outside the body, comprising a shaft (2) and an optical unit (11) arranged at a distal end of the shaft (2), which comprises an observation optical system for capturing the image of the object field, wherein the observation optical system has a first and a second stereo channel, each having an objective lens (20, 20') and each having at least one electronic image sensor (53, 53'), and the optical unit (11) is rotatable relative to the device about an axis of rotation (28) parallel to a viewing direction of the observation optical system, wherein the observation optical system comprises at least one filter (30, 30') which can be pivoted into an optical path of the observation optical system and pivoted out of the same, and the at least one filter (30, 30') is pivotable about a pivot axis (73, 73') directed parallel to the axis of rotation (28).

2. Device according to claim 1, **characterized in that** the observation optical system comprises the first filter (30) and a second filter (30'), wherein the first filter (30) can be pivoted into an optical path of the first stereo channel and pivoted out of the same, and the second filter (30') can be pivoted into an optical path of the second stereo channel and pivoted out of the same.

3. Device according to claim 2, **characterized in that** the at least one filter (30, 30') can be pivoted out of the optical path into a spatial region arranged laterally with respect to the optical paths of both stereo channels.

4. Device according to claim 2 or 3, **characterized in that** the first filter (30) can be pivoted into and out of a position between the objective lens (20) and the at least one image sensor (53, 53') of the first stereo channel, and the second filter (30') can be pivoted into and out of a position between the objective lens (20') and the at least one image sensor (53, 53') of the second stereo channel.

5. Device according to claim 3 or 4, **characterized in that** the spatial region is arranged in a direction transverse to a common plane of optical axes of the objective lenses (20, 20') and extends, in an orthogonal projection onto the common plane, over the same extent as the optical paths of the stereo channels.

6. Device according to one of claims 3 to 5, **characterized in that** the spatial region extends within a cylindrical housing of the rotatable optical unit, which encloses the optical paths of the stereo channels in a space-saving manner.

7. Device according to claim 5 or 6, **characterized in that** the pivot axis (73, 73') of the at least one filter (30, 30') is arranged in a region between the optical paths of the stereo channels or in such a way that a distance of the pivot axis (73, 73') from the axis of rotation (28) is smaller than a distance of the optical axes (25, 25') of the optical paths of the stereo channels from the axis of rotation (28).

8. Device according to one of claims 2 to 7, **characterized in that** the first filter (30) is offset relative to the second filter (30') in the axial direction, with respect to the axis of rotation (28).

9. Device according to one of claims 2 to 8, **characterized in that** the first filter (30) is pivotable about a first pivot axis (71) directed substantially parallel to the axis of rotation (28), and the second filter (30') is pivotable about a second pivot axis (71') adjacent to the first and likewise directed substantially parallel to the axis of rotation (28).

10. Device according to one of claims 2 to 8, **characterized in that** the first and the second filter (30, 30') are arranged on a common pivotable filter carrier.

11. Device according to one of claims 2 to 10, **characterized in that** the first and the second filter (30, 30') have identical spectral characteristics or different spectral characteristics.

12. Device according to one of the preceding claims, **characterized in that** the optical unit (11) comprises at least one sensor, in particular designed as a magnetic sensor (38) and/or as a proximity switch, for detecting a pivot position of the at least one filter (30, 30').

13. Device according to one of the preceding claims, **characterized in that** the observation optical system comprises a focusing device and/or the optical unit comprises a marking light source.

14. Device according to one of the preceding claims, **characterized in that** the first and the second stereo channel each comprise at least one further electronic image sensor (53, 53') and a beam splitter (41).

15. Device according to one of the preceding claims, **characterized in that** the optical unit (11) is of hermetically sealed design.

## Revendications

1. Dispositif destiné à la prise d'une image d'un champ d'objet sur un corps humain ou animal depuis l'extérieur du corps, comprenant une tige (2) et une unité optique (11) disposée à une extrémité distale de la tige (2), qui comprend une optique d'observation destinée à la prise de l'image du champ d'objet, l'optique d'observation présentant un premier et un second canal stéréoscopique, chacun comportant un objectif (20, 20') et chacun comportant au moins un capteur d'image électronique (53, 53'), et l'unité optique (11) étant rotative par rapport au dispositif autour d'un axe de rotation (28) parallèle à une direction de visée de l'optique d'observation, l'optique d'observation comprenant au moins un filtre (30, 30') pouvant être pivoté dans un trajet optique de l'optique d'observation et pivoté hors de celui-ci, et ledit au moins un filtre (30, 30') étant pivotable autour d'un axe de pivotement (73, 73') orienté parallèlement à l'axe de rotation (28).

2. Dispositif selon la revendication 1, **caractérisé en ce que** l'optique d'observation comprend le premier filtre (30) et un second filtre (30'), le premier filtre (30) pouvant être pivoté dans un trajet optique du premier canal stéréoscopique et pivoté hors de celui-ci, et le second filtre (30') pouvant être pivoté dans un trajet optique du second canal stéréoscopique et pivoté hors de celui-ci.

3. Dispositif selon la revendication 2, **caractérisé en ce que** ledit au moins un filtre (30, 30') peut être pivoté hors du trajet optique vers une région spatiale disposée latéralement par rapport aux trajets optiques des deux canaux stéréoscopiques.

4. Dispositif selon la revendication 2 ou 3, **caractérisé en ce que** le premier filtre (30) peut être pivoté entre l'objectif (20) et ledit au moins un capteur d'image (53, 53') du premier canal stéréoscopique et hors de cette position, et le second filtre (30') peut être pivoté entre l'objectif (20') et ledit au moins un capteur d'image (53, 53') du second canal stéréoscopique et hors de cette position.

5. Dispositif selon la revendication 3 ou 4, **caractérisé en ce que** la région spatiale est disposée dans une direction transversale à un plan commun des axes optiques des objectifs (20, 20') et s'étend, dans une projection perpendiculaire sur le plan commun, sur une même étendue que les trajets optiques des canaux stéréoscopiques.

6. Dispositif selon l'une quelconque des revendications 3 à 5, **caractérisé en ce que** la région spatiale s'étend à l'intérieur d'un boîtier cylindrique de l'unité optique rotative, lequel entoure les trajets optiques des canaux stéréoscopiques d'une manière peu encombrante.

7. Dispositif selon la revendication 5 ou 6, **caractérisé en ce que** l'axe de pivotement (73, 73') dudit au moins un filtre (30, 30') est disposé dans une région située entre les trajets optiques des canaux stéréoscopiques ou de telle sorte qu'une distance de l'axe de pivotement (73, 73') par rapport à l'axe de rotation (28) est inférieure à une distance des axes optiques (25, 25') des trajets optiques des canaux stéréoscopiques par rapport à l'axe de rotation (28).

8. Dispositif selon l'une quelconque des revendications 2 à 7, **caractérisé en ce que** le premier filtre (30) est décalé par rapport au second filtre (30') dans la direction axiale, par rapport à l'axe de rotation (28).

9. Dispositif selon l'une quelconque des revendications 2 à 8, **caractérisé en ce que** le premier filtre (30) est pivotable autour d'un premier axe de pivotement (71) orienté essentiellement parallèlement à l'axe de rotation (28), et le second filtre (30') est pivotable autour d'un second axe de pivotement (71') voisin du premier et également orienté essentiellement parallèlement à l'axe de rotation (28).

10. Dispositif selon l'une quelconque des revendications 2 à 8, **caractérisé en ce que** le premier et le second filtre (30, 30') sont disposés sur un support de filtre commun pivotable.

11. Dispositif selon l'une quelconque des revendications 2 à 10, **caractérisé en ce que** le premier et le second filtre (30, 30') présentent des caractéristiques spectrales identiques ou des caractéristiques spectrales différentes.

12. Dispositif selon l'une quelconque des revendications précédentes, **caractérisé en ce que** l'unité optique (11) comprend au moins un capteur, notamment conçu comme capteur magnétique (38) et/ou comme interrupteur de proximité, destiné à détecter une position de pivotement dudit au moins un filtre (30, 30').

13. Dispositif selon l'une quelconque des revendications précédentes, **caractérisé en ce que** l'optique d'observation comprend un dispositif de focalisation et/ou l'unité optique comprend une source lumineuse de marquage.

14. Dispositif selon l'une quelconque des revendications précédentes, **caractérisé en ce que** le premier et le second canal stéréoscopique comprennent chacun au moins un autre capteur d'image électronique (53, 53') ainsi qu'un séparateur de faisceau (41).

15. Dispositif selon l'une quelconque des revendications précédentes, **caractérisé en ce que** l'unité optique (11) est réalisée de manière hermétiquement étanche.
